# EUROPÄISCHE PATENTSCHRIFT

(11) **EP 4 252 659 B1**
(45) Veröffentlichungstag und Bekanntmachung des Hinweises auf die Patenterteilung: **10.09.2025**
(21) Anmeldenummer: 22165569.9
(22) Anmeldetag: 30.03.2022
(51) Int. Cl.: A61B 6/00, A61B 6/04

(54) **BEWEGUNGSSTEUERUNG FÜR EINE MEDIZINISCHE ANLAGE**
MOTION CONTROL FOR A MEDICAL INSTALLATION
COMMANDE DE DÉPLACEMENT POUR UNE INSTALLATION MÉDICALE

(43) Veröffentlichungstag der Anmeldung: 04.10.2023
(73) Patentinhaber: Siemens Healthineers AG, 91301 Forchheim (DE)
(72) Erfinder: Bößl, Sebastian, 95519 Schlammersdorf (DE); Griener, Michael, 95478 Kemnath (DE); Scherm, Markus, 95686 Fichtelberg (DE); Zeidler, Josef, 95615 Marktredwitz (DE)
(74) Vertreter: Siemens Healthineers Patent Attorneys

(56) Entgegenhaltungen:
- DE-A1- 102007 031 475
- DE-A1- 102007 032 533
- DE-A1- 102011 057 074
- DE-A1- 102013 219 744
- DE-B3- 102018 126 022

## Beschreibung

Die Erfindung betrifft eine Bewegungssteuerung für eine medizinische Anlage, speziell für eine bewegbare Komponente der medizinischen Anlage. Die Erfindung ermittelt einen Positionsabstand zweier Positionssensoren der Komponente und leitet aus diesem Positionsabstand eine manuelle Bedienkraft ab.

Bei medizinischen Anlagen wie medizinischen Bildgebungsanlagen, Radiographie, Computertomographie, Magnetresonanztomographie, Ultraschall oder dergleichen oder Anlagen zur medizinischen Behandlung wie bspw. Bestrahlung oder Intervention sind häufig bewegbare Komponenten vorgesehen, die während eines medizinischen Workflows bewegt werden. Zum Beispiel muss vor einer Bilddatenerfassung mittels Röntgentechnologie die Strahlungsquelle, der Röntgendetektor und der Patient bzw. die zu untersuchende Körperregion relativ zueinander positioniert werden. Dazu können bspw. die Patientenliege, auf welcher der Patient für die Untersuchung platziert ist, die Strahlungsquelle und/oder der Detektor individuell bewegt werden. Eine Verstellbewegung kann rein manuell, also mittels einer Bedienkraft, motorisch unterstützt oder vollautomatisch bzw. autonom erfolgen. Eine manuelle Verstellbewegung ist dabei besonders kostengünstig, da Antriebe und Führungsvorrichtungen eingespart werden können. Daneben bietet eine manuelle Verstellbewegung ein besonders natürliches und intuitives Bediengefühl für den Nutzer.

Bei großen und schweren Komponenten reicht jedoch eine manuelle Verstellkraft nicht mehr aus, um die Komponente komfortable zu bewegen. Besonders kraftaufwändig ist es dann, die Komponente aus einer Ruhelage in Bewegung zu versetzen. Dies gilt zum Beispiel, wenn ganze medizinische Anlagen, bspw. eine mobile C-Bogen-Anlage, bewegt werden müssen. Insbesondere ist dies auch bei einer Patientenliege der Fall. In Abhängigkeit des Patientengewichts ist hier eine manuell handhabbare Gesamtmasse, diese liegt im Bereich von 100 kg - 200 kg, leicht überschritten.

Mit hochwertigen und in der Regel hochpreisigen Führungssystemen können heute hohe Bedienkräfte reduziert werden.

Alternativ werden an der bewegbaren Komponente Bedienelemente integriert, die mit Kraftsensoren ausgerüstet sind. Ein Sensorsignal entsprechend einer detektierten Bedienkraft wird zur Steuerung eines aktiven, motorischen Antriebs verwendet. Dabei geht einerseits das natürliche Bediengefühl verloren, denn die Hauptlast für die Komponentenbewegung wird vom motorischen Antrieb geleistet. Daneben ist das Bedienelement inklusive Kraftsensorik örtlich festgelegt. Eine Verstellbewegung kann nur über Berührung dieses Bedienelements initiiert werden.

Die Druckschrift DE 10 2007 032 533 A1 beschreibt ein Verfahren und ein entsprechendes Gerät zur Vermeidung von Kollisionen von Teilen eines medizinischen Diagnose- und/oder Therapiegeräts mit externen Objekten. Das Verfahren beinhaltet die Auswertung von Daten eines Abstands- und/oder Näherungssensors, um zwischen tatsächlichen Kollisionen und gewollten Annäherungen der Geräteteile zu unterscheiden. Dabei werden Informationen über die Geometrie des Geräts, den Anbringungsort des Sensors und Echtzeit-Positionsdaten genutzt. Kalibrationsdaten können verwendet werden, um erwartete Sensorwerte während der Bewegung zu bestimmen und mit den aktuellen Werten zu vergleichen. Bei Überschreitung eines Schwellwerts werden die Bewegungen des Geräts eingeschränkt oder gestoppt oder es wird ein Warnsignal ausgegeben. Das Gerät selbst umfasst einen beweglichen Komponententräger, eine Systemsteuerungseinheit, Abstands-/Näherungssensoren, Steuermittel und Auswertemittel. Die Auswertemittel analysieren die Sensorwerte, um festzustellen, ob eine Eigenbewegung des Geräts vorliegt, und die Steuermittel nutzen diese Informationen zur weiteren Steuerung des Komponententrägers. Verschiedene Ausführungen des Geräts sind möglich, darunter solche mit mehrachsigen Knickarmrobotern. Ein Sensor-Kollisionsrechner kann in die Komponententräger-Steuerungseinheit integriert sein, um eine schnelle Reaktion auf Kollisionssituationen zu ermöglichen. Ein übergeordneter Kollisionsrechner kann vorhanden sein, wenn mehrere Geräteteile und Sensoren beteiligt sind. Speichermittel können verwendet werden, um vorberechnete oder gemessene Daten zu speichern und den Auswerteprozess zu verbessern.

Demgegenüber ist es Aufgabe der vorliegenden Erfindung, alternative Mittel bereit zu stellen, die eine nutzerfreundliche, kosteneffiziente und flexible Bewegungssteuerung für eine medizinische Anlage bzw. eine ihrer Komponenten erlauben. Diese Aufgabe wird gelöst durch ein Verfahren und ein entsprechendes System zur Steuerung einer Bewegung einer Komponente einer medizinischen Anlage sowie eine entsprechende medizinische Anlage gemäß den unabhängigen Ansprüchen. Bevorzugte und/oder alternative, vorteilhafte Ausgestaltungsvarianten sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Die vorliegende Erfindung betrifft in einem ersten Aspekt ein Verfahren zur Steuerung einer Bewegung einer Komponente einer medizinischen Anlage, wobei die Komponente bewegbar ist und die bewegbare Komponente als elastisches, reversibel verformbares Element ausgebildet ist, und wobei die elastische Verformung der Komponente durch bzw. im Wesentlichen durch eine manuell eingebrachte Bedienkraft eines Nutzers hervorgerufen wird. Das Verfahren umfasst eine Vielzahl von Schritten. In einem ersten Schritt erfolgt ein Erfassen eines ersten Positionsparameters mittels eines ersten an der Komponente angeordneten Positionssensors. In einem zweiten Schritt erfolgt ein Erfassen eines zweiten Positionsparameters mittels eines zweiten an der Komponente angeordneten Positionssensors. In einem dritten Schritt erfolgt ein Ermitteln eines Positionsabstands für die Positionssensoren basierend auf dem ersten und dem zweiten Positionsparameter mittels einer Steuereinheit, wobei der Positionsabstand der Abstand zwischen den beiden Positionssensoren entlang einer Raumachse ist. In einem weiteren Schritt erfolgt ein Erzeugen eines Steuersignals für eine Antriebseinheit der Komponente basierend auf dem ermittelten Positionsabstand mittels der Steuereinheit.

Entsprechend ist ein zweiter Aspekt der vorliegenden Erfindung auf ein System zur Steuerung einer Bewegung einer Komponente einer medizinischen Anlage gerichtet. Das System umfasst neben der bewegbaren Komponente, welche als elastisches, reversibel verformbares Element ausgebildet ist, und wobei die elastische Verformung der Komponente durch bzw. im Wesentlichen durch eine manuell eingebrachte Bedienkraft eines Nutzers hervorgerufen wird, einen ersten an der Komponente angeordneten Positionssensor, eingerichtet zum Erfassen eines ersten Positionsparameters, einen zweiten an der Komponente angeordneten Positionssensor, eingerichtet zum Erfassen eines zweiten Positionsparameters sowie eine Steuereinheit. Diese ist eingerichtet zum Ermitteln eines Positionsabstands für die Positionssensoren basierend auf dem ersten und dem zweiten Positionsparameter, wobei der Positionsabstand der Abstand zwischen den beiden Positionssensoren entlang einer Raumachse ist, und zum Erzeugen eines Steuersignals für eine Antriebseinheit der Komponente basierend auf dem ermittelten Positionsabstand.

Ein dritter Aspekt der vorliegenden Erfindung betrifft eine medizinische Anlage. . In Ausführungen bildet die medizinische Anlage selbst als Ganzes die bewegbare Komponente. Die medizinische Anlage umfasst weiter eine Antriebseinheit sowie ein erfindungsgemäßes System zur Steuerung einer Bewegung der Komponente. Die Antriebseinheit kann in weiteren Ausführungen der Erfindung auch als Bestandteil der bewegbaren Komponente ausgebildet sein.

Die medizinische Anlage kann als medizinische Bildgebungsanlage aber auch als Anlage zur medizinischen Behandlung oder Intervention ausgebildet sein. Bspw. handelt es sich bei der medizinischen Anlage um eine Radiographie-Anlage, bei der mittels Röntgenstrahlung vermehrt digitale Röntgenbildaufnahmen erzeugt werden. Es kann sich aber auch um eine Computertomographie-Anlage, eine Magnetresonanztomographie-Anlage oder dergleichen handeln. Die medizinische Anlage umfasst eine, bevorzugt mehrere bewegbare Komponenten. Eine bewegbare Komponente bezeichnet dabei eine Einheit der Anlage, die relativ zu anderen Einheiten der Anlage bzw. zur Umgebung der Anlage bewegt werden kann. Die bewegbare Komponente kann manuell, motorisch mittels der Antriebseinheit unterstützt oder voll motorisch bewegt werden. Die Bewegung bezieht sich in bevorzugten Ausführungen der Erfindung auf eine Translation entlang einer Raumachse. Eine bewegbare Komponente kann aber auch translatorisch entlang mehrerer Raumachsen verstellbar sein, sodass sich die Gesamtbewegung der Komponente als Überlagerung der Einzeltranslationen ergibt.

Vorteilhaft ist das erfindungsgemäße System zur Steuerung einer Bewegung in die medizinische Anlage integriert. Alternativ können zumindest einzelne Einheiten des Systems, insbesondere die Steuereinheit oder Submodule derselben, entfernt bzw. abgelegen angeordnet sein. Das erfindungsgemäße System kann ausgebildet sein, insbesondere den Schritt des Ermittelns eines Positionsabstands sowie den Schritt des Erzeugens eines Steuersignals, aber auch das gesamte erfindungsgemäße Verfahren, für eine medizinische Anlage durchzuführen.

Eine bewegbare Komponente kann in Ausführungen der Erfindung als Stativ, bspw. ein Rasterwandgerät oder ein Deckenstativ, oder als eine Patientenliege bzw. das auf dieser angeordnete Liegenbrett ausgebildet sein. Rasterwandgerät und Deckenstativ dienen typischerweise zur Befestigung und Positionierung bildgebender Einheiten wie Röntgenstrahlenquelle und/oder Röntgendetektor. Die Patientenliege dient zur Aufnahme bzw. Lagerung und Positionierung eines Patienten.

Eine erfindungsgemäße Antriebseinheit ist in bevorzugter Ausführung als Linearantrieb ausgebildet. Dieser umfasst bevorzugt einen Elektromotor, bzw. einen rotatorischen Servomotor, der eine Welle rotatorisch antreibt und deren Rotation über ein entsprechendes Getriebe in eine Linearbewegung der Komponente übersetzt wird. Alternative Antriebskonzepte sind ebenfalls denkbar und im Sinne der Erfindung. Die Antriebseinheit umfasst in Ausführungen auch eine Hilfsantrieb, der ausgebildet ist, eine richtungsabhängige Reibungskompensation zu bewirken.

Die Antriebseinheit kann als Bestandteil der Komponente bei einer Verstellbewegung mitbewegt werden oder zumindest teilweise relativ zu einer weiteren Einheit der medizinischen Anlage stationär ausgebildet sein.

Erfindungsgemäß sind zwei voneinander verschiedene und unabhängige Positionssensoren vorgesehen. Jeder Positionssensor ist ausgebildet, eine Positionsinformation entsprechend seiner räumlichen Lage zu erfassen und basierend auf der Positionsinformation einen weiter verarbeitbaren Positionsparameter zu generieren, der an eine Steuereinheit des erfindungsgemäßen Systems übergeben wird. Insofern beschreibt das Erfassen eines Positionsparameters auch ein Erfassen durch bzw. ein Übertragen des Positionsparameters von einem Positionssensor an die Steuereinheit.

Die Positionsparameter sind in bevorzugter Ausführung als Positionsangaben zu verstehen, die in Bezug auf dasselbe Koordinatensystem, bspw. das Anlagen-Koordinaten-System angegeben werden. Hierbei handelt es sich typischerweise um ein dreidimensionales Koordinatensystem.

Die Positionsparameter sind in besonders bevorzugter Ausführung eindimensional.

Die Erfindung geht davon aus, dass die beiden Positionssensoren voneinander entfernt, insbesondere entlang einer Raumachse voneinander entfernt, an der Komponente angeordnet bzw. integriert sind und sich die erfassten Positionsparameter immer unterscheiden.

Als nächstes ermittelt die Steuereinheit einen Positionsabstand entlang einer Raumachse. Es wird also ermittelt, wie weit beide Positionssensoren voneinander beabstandet sind. Der ermittelte Positionsabstand umfasst dabei einen betragsmäßigen Abstand, umfasst aber über sein Vorzeichen auch eine Information bezüglich der relativen Lage der Positionssensoren zueinander und damit auch eine Richtungsangabe zu der manuellen Bedienkraft. Die Erfindung geht weiter davon aus, dass der ermittelte Positionsabstand eine von einer in die bewegbare Komponente eingetragenen, entlang der Raumachse wirkenden, manuellen Bedienkraft abhängige Größe ist. Mit anderen Worten geht die Erfindung davon aus, dass sich der ermittelte Positionsabstand in Richtung der Raumachse mit der Bedienkraft eines Nutzers verändert. Diese Vorgehensweise beruht darauf, dass die bewegbare Komponente als elastisches, also reversibel verformbares Element ausgebildet ist, wobei die elastische Verformung der Komponente durch bzw. im Wesentlichen durch die manuell eingebrachte Bedienkraft des Nutzers hervorgerufen wird.

Anhand des so ermittelten Positionsabstandes erlaubt die Erfindung einen Rückschluss auf die auf die Komponente wirkende manuelle Bedienkraft. Entsprechend wird in einem weiteren Schritt ein Steuersignal für die Antriebseinheit basierend auf dem ermittelten Positionsabstand erzeugt. In bevorzugten Ausführungen der Erfindung umfasst das Erzeugen des Steuersignals, dem Positionsabstand eine der manuellen Bedienkraft entsprechende Verstellkraft zuzuordnen. In anderen Ausführungen wird der Positionsabstand direkt genutzt, um ein Steuersignal entsprechend der zugehörigen Verstellkraft zu erzeugen. Der Zwischenschritt entfällt in diesen Ausführungen.

In bevorzugter Ausführung sind die den Positionsabständen zugewiesenen Verstellkräfte spezifisch für die bewegbare Komponente. Damit trägt die Erfindung vorteilhaft der Tatsache Rechnung, dass jede Komponente ein unterschiedliches elastisches Verformungsverhalten aufweisen kann. Das elastische Verformungsverhalten ist dabei bspw. von der Grundform, den verbauten Materialien und/oder der Größe der bewegbaren Komponente abhängig. Eine kompakte, schwere Komponente wird bspw. eine höhere Steifigkeit aufweisen als eine längliche, dünne bzw. leichte Komponente.

Die vorliegende Erfindung ersetzt vorteilhaft einen Kraftsensor, um eine manuelle Bedienkraft bzgl. ihrer Richtung und/oder ihres Betrags zu erfassen. Dadurch entfallen die Kosten für den Kraftsensor. Zudem ermöglicht es die Erfindung, dass eine manuelle Bedienkraft an beliebiger Stelle der bewegbaren Komponente eingebracht werden kann. Dies entspricht einer Vorgehensweise, wie sie auch für eine rein manuelle Verstellbewegung angewandt wird. Da kein Kraftsensor implementiert werden muss, kann der Nutzer nun an jeder beliebigen Stelle der Komponente die manuelle Bedienkraft einbringen und ist nicht auf das Bedienelement umfassend den Kraftsensor beschränkt.

In Ausführungen sind der erste und der zweite Positionssensor derart an der Komponente angeordnet, dass sie einen maximalen Ruheabstand entlang einer Raumachse zueinander aufweisen.

Der Ruheabstand beschreibt den Abstand beider Sensoren, wenn die Komponente unbewegt ist und keine Kraft auf die Komponente wirkt.

Je weiter die beiden Positionssensoren voneinander beabstandet sind, umso genauer lässt sich das elastische Verformungsverhalten der Komponente mittels des Positionsabstands darstellen. In weiteren Ausführungen ist jedoch eine Anordnung der Positionssensoren innerhalb des Antriebsstrangs besser geeignet. Bspw. ist der erste Positionssensor direkt im oder am Antrieb, also der Antriebseinheit verbaut, wobei der zweite Positionssensor im oder am Abtrieb, vorteilhaft dann an einem hinteren Teil des Abtriebs, angeordnet. Bspw. ist der zweite Positionssensor dann an der Abtriebswelle der Antriebseinheit, also insbesondere hinter einem Getriebe der Antriebseinheit angeordnet.

Besonders vorteilhaft sind Ausführungen der Erfindung, bei welcher zum Erfassen der Positionsparameter Positionssensoren verwendet werden, die ohnehin in der medizinischen Anlage bzw. der bewegbaren Komponente verbaut sind, bspw. die Positionssensoren, die eingesetzt werden, um einen erstfehlersicheren Betrieb der medizinischen Anlage zu gewährleisten. Derart können zusätzliche Bauteile sowie Zusatzkosten entfallen.

Entsprechend sehen besonders vorteilhafte Ausbildungen der Erfindung vor, dass der erste Positionssensor bspw. als Drehgeber der Antriebseinheit ausgebildet ist. Der Drehgeber ist fester Bestandteil der Antriebseinheit, der zur korrekten Ausübung der Motorfunktion kontinuierlich die Winkellage des Motors erfasst. Über die Winkellage und ein Überwachen der Motorumdrehungen kann so eine Positionsinformation in Form eines Positionsparameters bereitgestellt werden.

Der zweite Positionssensor oder in alternativen Ausführungen der Erfindung beide Positionssensoren können auf beliebige und an sich bekannte andere Weise ausgebildet sein. Bspw. kann ein Positionssensor als Lasersensor, induktiver Sensor, Ultraschall-Sensor, kapazitiver Sensor oder dergleichen ausgebildet sein.

Die Steuereinheit ist ausgebildet, einen Positionsabstand anhand der Positionsparameter zu ermitteln und ein Steuersignal zur Bewegungssteuerung der Komponente basierend auf dem Positionsabstand zu erzeugen. Der Positionsabstand ist repräsentativ für eine auf die Komponente eingebrachte manuelle Bedienkraft. Daher ist das Steuersignal geeignet, die Komponente entsprechend der manuellen Bedienkraft zu steuern. Die Steuereinheit umfasst eine Schnittstelle zur Eingabe bzw. zum Erfassen der Positionsparameter und zur Ausgabe des Steuersignals an die Antriebseinheit. Die Schnittstelle kann bevorzugt als integrale Ein- und Ausgabeschnittstelle ausgebildet sein.

Die Steuereinheit kann in Ausführungen der Erfindung als eine oder mehrere zentrale und/oder dezentrale Rechenmodule ausgebildet sein. Die Rechenmodule können jeweils einen oder mehrere Prozessoren aufweisen. Ein Prozessor kann als zentrale Verarbeitungseinheit (CPU/GPU) ausgebildet sein. Die Steuereinheit kann insbesondere einen Motorcontroller der Antriebseinheit umfassen. In weiteren Ausführungen ist der Motorcontroller als Teil der Antriebseinheit ausgebildet. Weiter umfasst die Steuereinheit ein Auswertemodul bzw. eine Auswerteeinheit, welches die Positionsparameter verarbeitet und basierend auf dem ermittelten Positionsabstand ein Steuersignal erzeugt.

In bevorzugten Implementierungen ist die Steuereinheit wiederum als Teil oder Modul einer zentralen Recheneinheit der medizinischen Anlage implementiert. In Implementierungen kann die Steuereinheit als Submodul der zentralen Recheneinheit ausgebildet sein oder umgekehrt. Alternativ kann die Steuereinheit als lokaler oder Cloud-basierter Verarbeitungsserver implementiert sein. Ferner kann die Steuereinheit ein oder mehrere virtuelle Maschinen umfassen.

Die Schnittstelle der Steuereinheit kann allgemein zum Datenaustausch zwischen Positionssensoren und Antriebseinheit und der Steuereinheit und/oder zum Datenaustausch von Modulen der Steuereinheit untereinander ausgebildet sein. Die Schnittstelle kann insofern in Form von einer oder mehreren einzelnen Datenschnittstellen implementiert sein, welche ein Hardware- und/oder Software-Interface, einen Daten-Bus, bspw. einen PCI-Bus, eine USB-Schnittstelle, eine Fire-Wire-Schnittstelle, eine ZigBee- oder eine Bluetooth-Schnittstelle aufweisen können.

Insbesondere das Auswertemodul und der Motorcontroller sind über einen Daten-Bus miteinander verbunden. Die Schnittstelle kann ferner eine Schnittstelle eines Kommunikationsnetzwerks aufweisen, wobei das Kommunikationsnetzwerk ein Local Area Network (LAN), beispielsweise ein Intranet oder ein Wide Area Network (WAN) aufweisen kann. Entsprechend können die ein oder mehreren Datenschnittstellen eine LAN-Schnittstelle oder eine Wireless LAN-Schnittstelle (WLAN oder Wi-Fi) aufweisen.

Besonders bevorzugt ist die Steuereinheit des Systems ausgebildet, eine Auswertung von Positionsparametern für verschiedene Raumachsen vorzunehmen. Hierbei werden für die verschiedenen Raumachsen jeweils zwei Positionsparameter erfasst, Positionsabstände ermittelt und Steuersignale für eine Bewegungssteuerung entlang der mehrere Raumachsen erzeugt. Entsprechend kann in Ausführungen des erfindungsgemäßen Systems ein Paar erstem und zweitem Positionssensor für jede Raumachse umfassen. Gleichzeitig ist die Steuereinheit dann eingerichtet, einen Positionsabstand für die Positionssensor-Paare jeder Raumachse und ein jeweiliges Steuersignal für die Antriebseinheit jeder Raumachse basierend auf dem jeweiligen Positionsabstand zu erzeugen.

Entsprechend umfasst das System in manchen Ausführungen wenigstens eine Antriebseinheit pro Raumachse.

Besonders vorteilhaft da Bauteile sparend ist es, wenn mehrdimensionale Positionssensoren verwendet werden, die Positionsparameter bezüglich mehrerer Raumachsen liefern können. Bei der Anordnung mehrdimensionaler Positionssensoren muss vorteilhaft ein Kompromiss gefunden werden, sodass für alle erfassten Raumachsen das elastische Verformungsverhalten über die Positionsabstände darstellbar ist.

In vorteilhafter Ausführung umfasst das Verfahren ein Zuordnen einer auf die Komponente wirkenden Verstellkraft zu dem ermittelten Positionsabstand, wobei das Erzeugen des Steuersignals für die Antriebseinheit basierend auf der zugeordneten Verstellkraft erfolgt.

Hierzu kann ein Datenaustausch zwischen der Steuereinheit und einer internen oder externen Speichereinheit umfasst sein, in der bspw. in Form einer Look-Up Tabelle verschiedenen Positionsabständen zu der bewegbaren Komponente insbesondere für das Auswertemodul bzw. die Auswerteinheit abrufbar Verstellkräfte zugewiesen sind. Alternativ oder zusätzlich kann vorgesehen sein, dass das Auswertemodul basierend auf vorhandenen Datenpaaren einen Inter- oder Extrapolationsschritt ausführt, um eine zugehörige Verstellkraft abzuleiten.

Die Speichereinheit kann nun eine weitere Look-Up Tabelle umfassen, die einen Zusammenhang zwischen der Verstellkraft und einer entsprechenden Steuerinformation herstellt, basierend auf welcher das Steuersignal abgeleitet wird. Die Steuerinformation liegt bspw. als der Verstellkraft entsprechende Drehmoment- Geschwindigkeit- oder Positionsvorgabe für die Antriebseinheit vor.

In anderen Ausführungen kann die Speichereinheit bspw. mittels einer Look-Up Tabelle einen direkten Zusammenhang zwischen dem ermittelten Positionsabstand und einer entsprechenden Steuerinformation herstellen.

Besonders vorteilhaft umfasst das Erzeugen des Steuersignals für die Antriebseinheit, eine Drehmomentvorgabe für die Antriebseinheit basierend auf dem Betrag der zugeordneten Verstellkraft zur motorischen Kraftunterstützung zu bestimmen. Eine Drehmomentvorgabe bietet ggü. anderen Steuerinformationen für den Nutzer den Vorteil eines authentischen Bediengefühls. Mit anderen Worten richtet sich der Wert der Drehmomentvorgabe nach dem Betrag des Positionsabstands. In weiteren Ausführungen kann die Steuerinformation aber auch in Form eine Positions- und/oder einer Geschwindigkeitsvorgabe für die Antriebseinheit vorliegen. Dies kann bspw. wieder von der Art der Komponente bzw. der Art der Antriebseinheit vorgegeben sein.

Bevorzugt umfasst der ermittelte Positionsabstand auch eine Richtungsinformation in Bezug auf die manuelle Bedienkraft. Insofern ermöglicht die Erfindung nicht nur eine Drehmoment-vorgabe, sondern auch eine Vorgabe der Bewegungsrichtung für die Antriebseinheit entsprechend der Richtung der manuellen Bedienkraft mittels Steuersignal. Bevorzugt kann neben der Drehmomentvorgabe auch die Drehrichtung für die Antriebseinheit als Steuerinformation erzeugt werden.

Um verlässlich anhand des Positionsabstandes auf die manuelle Bedienkraft rückschließen zu können, müssen die Positionssensoren zeitgleich ihre Positionsparameter erfassen und an die Steuereinheit übertragen. Entsprechend ist in Ausführungen der Erfindung vorgesehen, dass das Erfassen des ersten und des zweiten Positionsparameters gleichzeitig erfolgt. Gleichzeitig soll im Sinne der Erfindung verstanden werden als ,mit kleinstmöglichem zeitlichem Versatz'.

Bevorzugt werden zu diesem Zweck beide Positionssensoren mit dem Motorcontroller verbunden und über diesen ausgelesen. Der Motorcontroller wird erfindungsgemäß derart konfiguriert, dass er den ersten und den zweiten Positionsparameter in ein und demselben Datenpaket, auch Telegramm genannt, über den Daten-Bus zur Weiterverarbeitung an das Auswertemodul der Steuereinheit übermittelt. Derart wird verhindert, dass ein Zeitversatz durch das Versenden von mehreren Datenpaketen hintereinander über den Daten-Bus entstehen kann.

In bestimmten Situationen kann es sein, dass eine manuelle Bedienkraft auf die Komponente aufgebracht wird, während diese sich bereits bewegt, bspw. um eine Richtungsänderung oder eine Bewegungsbeschleunigung zu initiieren, wobei die aktuelle Bewegung auf einen motorischen Krafteintrag zurückzuführen ist. Entsprechend umfasst in bevorzugter Implementierung das erfindungsgemäße Verfahren ein Erfassen eines Beschleunigungsparameters der Antriebseinheit zeitgleich zum Erfassen des ersten und des zweiten Positionsparameters. Zudem wird der motorische Krafteintrag der Antriebseinheit basierend auf dem Beschleunigungsparameter, ermittelt und damit die Verstellkraft korrigiert.

In bevorzugter Ausführung erfolgt dabei das Korrigieren der Verstellkraft durch Subtraktion des ermittelten motorischen Krafteintrags.

Diese Vorgehensweise beruht auf der Annahme, dass derjenige Kraftanteil der Verstellkraft, also die korrigierte Verstellkraft, der nicht durch die Antriebseinheit eingebracht wird, allein auf die manuelle Bedienkraft des Nutzers zurückgeht.

Zur Berechnung des motorischen Krafteintrags wird über den Beschleunigungsparameter, eine Größe, anhand derer sich ein Wert für eine aktuelle Beschleunigung der Antriebseinheit ermitteln lässt, die wirkende Beschleunigung erfasst und mit der bewegten Masse der Komponente multipliziert. Die Komponentenmasse kann dabei der Steuereinheit bzw. dem Auswertemodul als fester Masseparameter bspw. von der eingangs beschriebenen Speichereinheit bereitgestellt werden.

Die beschriebene Vorgehensweise ermöglicht es, die Verstellkraft besser an die tatsächliche manuelle Bedienkraft anzupassen. Das Steuersignal wird in dieser Ausführung basierend auf der korrigierten Verstellkraft erzeugt.

In Ausführungen umfasst das erfindungsgemäße System also auch einen Beschleunigungssensor, der bevorzugt in die Antriebseinheit integriert ist bzw. mit dieser zusammenwirkt. Der Beschleunigungssensor kann bspw. als an sich bekannter MEMS-Sensor, als Dehnungsmesstreifen oder als Piezo-elektrischer Beschleunigungssensor ausgebildet sein.

Vorteilhaft wird auch der Beschleunigungsparameter zeitgleich mit dem ersten und zweiten Positionssensor erfasst und über den Motorcontroller mit demselben Datenpaket an das Auswertemodul der Steuereinheit übertragen, um die Gleichzeitigkeit zu gewährleisten.

Alternative Ausgestaltungen, in denen der Positionsabstand direkt in ein Steuersignal entsprechend der dazugehörigen Verstellkraft umgesetzt wird, können basierend auf dem wie oben beschrieben ermittelten motorischen Krafteintrag auch eine motorisch bedingte Positionsdifferenz ableiten und damit eine Korrektur des Positionsabstands vornehmen (ebenfalls durch Subtraktion). Das Steuersignal für die Antriebseinheit wird dann basierend auf dem korrigierten Positionsabstand erzeugt.

Wie eingangs erwähnt, umfassen die Positionsparameter eine Information bezüglich der Wirkrichtung der manuellen Bedienkraft, sodass anhand des ermittelten Positionsabstands die manuelle Bedienkraft nicht nur dem Betrag nach, sondern auch in ihrer Richtung (vor oder zurück entlang der Raumachse) bestimmbar ist. Im Wesentlichen wird die Wirkrichtung dabei durch das Vorzeichen des ermittelten Positionsabstandes definiert. In Situationen, in denen die Verstellkraft dem Betrage nach nicht verlässlich bestimmt werden kann, kann das Erzeugen des Steuersignals für die Antriebseinheit zumindest umfassen, basierend auf dem Vorzeichen des Positionsabstands eine Drehmomentvorgabe für die Antriebseinheit, insbesondere den Hilfsantrieb, zur richtungsabhängigen Reibungskompensation zu erzeugen. Die Antriebseinheit wird hier folglich derart angesteuert, dass sie eine motorische Antriebskraft in die entsprechende Richtung auf die Komponente ausübt, die zur Überwindung der massebedingten Haftreibung ausreicht. Auch hierzu kann das Auswertemodul bzw. die Auswerteinheit wieder auf den abrufbar hinterlegten Masseparameter der Komponente zugreifen. Alternativ kann in der Speichereinheit auch direkt der für die Komponentenmasse spezifische, erforderliche Drehmoment hinterlegt sein.

In alternativen Ausführungen kann in Abhängigkeit der Ausgestaltung der Antriebseinheit auch eine richtungsabhängige Positions- oder Geschwindigkeitsvorgabe für die Antriebseinheit erzeugt werden.

Um die medizinische Anlage kontinuierlich zu überwachen und auf einen manuellen Krafteintrag hin zu prüfen, wird in besonders bevorzugten Implementierungen das erfindungsgemäße Verfahren wiederholt und besonders bevorzugt mit einem zeitlichen Abstand von bspw. 5 ms oder 10 ms ausgeführt. Andere Zeitintervalle sind ebenfalls möglich und im Sinne der Erfindung. Das gewählte Zeitintervall kann insbesondere wieder von der bewegbaren Komponente und bspw. deren typischem Bewegungsverhalten abhängen- Mit anderen Worten werden regelmäßig Positions- und ggf. Beschleunigungsparameter von den entsprechenden Sensoren abgerufen und mittels Steuereinheit ausgewertet. Derart können Änderungen der manuellen Bedienkraft (in Bezug auf Betrag und/oder Richtung) im Wesentlichen ohne zeitlichen Verzug registriert und Anpassungen in Bezug auf die motorische Bewegungssteuerung bzw. eine Bewegungsregelung vorgenommen werden.

Weitere Ausführungen des erfindungsgemäßen Verfahrens sehen vor, dass das Ermitteln des Positionsabstands umfasst, einen vorab definierten, für die bewegbare Komponente der medizinischen Anlage spezifischen Offset repräsentierend eine nicht-elastische Verformbarkeit der Komponente zu subtrahieren, wenn sich das Vorzeichen des Positionsabstands zwischen zwei Verfahrensdurchläufen ändert.

Diese Vorgehensweise beruht auf der Erkenntnis, dass die bewegbare Komponente durch einen (manuellen) Krafteintrag nicht nur elastisch, sondern auch nicht-elastisch verformt wird. Diese nicht-elastische Verformung kann bspw. auf Bauteiltoleranzen, ein Lagerspiel zwischen zwei ineinandergreifenden Bauteilen der Komponente oder dergleichen, insbesondere auf Toleranzen der Antriebseinheit basieren, sodass bei Krafteinwirkung Bauteile der Komponente relativ zueinander bewegbar sind. Diese nicht elastische Verformung bspw. in Form einer Längenänderung der Komponente bzw. einer Positionsänderung eines des Positionssensoren in der betrachteten Raumachse tritt immer dann auf, wenn die Bewegungsrichtung der Komponente geändert wird, sich also das Vorzeichen des Positionsabstands ändert. Sie ist nicht auf die manuelle Bedienkraft selbst zurückzuführen. Die nicht-elastische Verformung ist wiederum spezifisch für den Aufbau bzw. die einzelnen Bestandteile der bewegbaren Komponenten und kann vorab experimentell ermittelt und als fester Längenparameter für das Auswertemodul hinterlegt werden.

In dieser Ausführung umfasst das Verfahren daher vorteilhaft einen Schritt eines Vorzeichenvergleichens des Positionsabstandes zwischen zwei Verfahrensdurchläufen. Zudem umfasst das Verfahren in dieser Ausführung auch eine Überwachung der Bewegungsrichtung der Antriebseinheit. Vorzeichenvergleich und Überwachung der Bewegungsrichtung werden bevorzugt für jeden der Verfahrensdurchläufe, also kontinuierlich ausgeführt. Ändert sich das Vorzeichen, wird bei der Ermittlung des Positionsabstands pauschal bevorzugt die Hälfte des Längenparameters unter Berücksichtigung der Bewegungsrichtung zu einem der Positionsparameter oder dem Positionsabstand hinzuaddiert oder davon abgezogen. Auch dadurch wird die die tatsächlich eingebrachte manuelle Bedienkraft genauer über den ermittelten Positionsabstand abgebildet.

Die Erfindung betrifft ferner ein Computerprogramm mit Programmcode, um das erfindungsgemäße Verfahren zur Steuerung einer Bewegung einer Komponente einer medizinischen Anlage durchzuführen, wenn das Computerprogramm auf einem Computer ausgeführt wird.

Die Erfindung betrifft ferner einen computerlesbarer Datenträger mit Programmcode eines Computerprogramms, um das erfindungsgemäße Verfahren zur Steuerung einer Bewegung einer Komponente einer medizinischen Anlage durchzuführen, wenn das Computerprogramm auf einem Computer ausgeführt wird. Vorteilhaft kann insbesondere das Ermitteln des Positionsabstands bzw. das Erzeugen des Steuersignals basierend auf dem Positionsabstand auf einem Computer, beispielsweise einer Recheneinheit der medizinischen Anlage, ausgeführt werden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: ein schematisches Ablaufdiagramm eines erfindungsgemäßen Verfahrens in einer Ausführung,
- FIG 2: eine Detailansicht einer medizinischen Anlage umfassend ein erfindungsgemäßes System zur Steuerung in einer Ausführung, und
- FIG 3: ein Diagramm darstellend einen zeitlichen Verlauf des Positionsabstands im Zusammenhang mit der ermittelten Verstellkraft, einer motorischen Beschleunigung und einer Bewegungsgeschwindigkeit der Komponente in einer Ausführung der Erfindung.

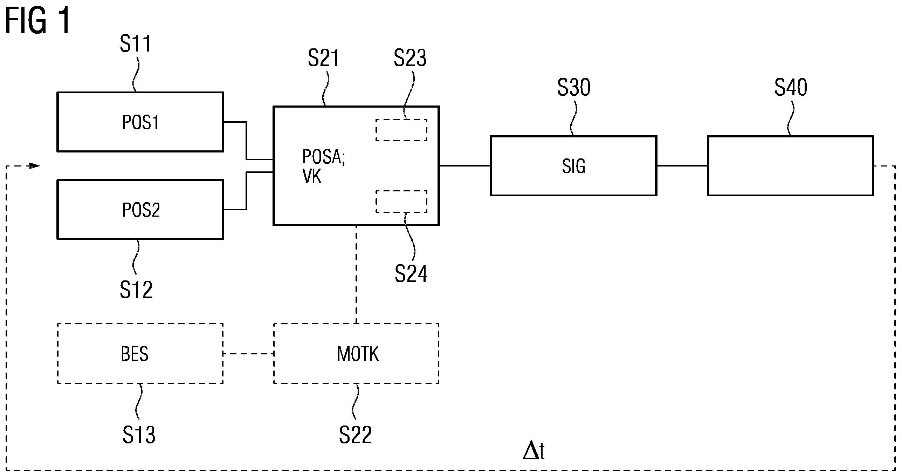

**Figur 1** zeigt ein schematisches Ablaufdiagramm eines erfindungsgemäßen Verfahrens in einer Ausführung. Das Verfahren dient zur Steuerung einer Bewegung, insbesondere einer Verstellbewegung einer Komponente 30 einer medizinischen Anlage 10.

In einem ersten Schritt erfolgt ein Erfassen S11 eines ersten Positionsparameters POS1 mittels eines ersten an der Komponente 30 angeordneten Positionssensors PS1.

In einem zweiten Schritt erfolgt ein Erfassen S12 eines zweiten Positionsparameters POS2 mittels eines zweiten an der Komponente angeordneten Positionssensors PS2.

Beide Positionsparameter POS1 und POS 2 sind dergestalt, dass anhand ihrer Werte auf eine Lage und/oder eine Position in Bezug zu einem Referenz-Koordinatensystem, bevorzugt das Koordinatensystem der medizinischen Anlage 10 geschlossen werden kann. Im einfachsten Fall geben die Positionsparameter POS1 und POS2 die Position des zugehörigen Positionssensors PS1, PS2 direkt an.

Ein weiterer Schritt S21 ist auf ein Ermitteln eines Positionsabstands POSA für die Positionssensoren PS1, PS2 basierend auf dem ersten und dem zweiten Positionsparameter POS1, POS2 mittels einer Steuereinheit SE gerichtet. Beide Positionssensoren PS1, PS2 sind dadurch gekennzeichnet, dass die an der beweglichen Komponente 30 angeordnet sind, aber jeweils an unterschiedlichen Positionen (zumindest in Bezug auf eine Raumachse). Die Erfindung geht davon aus, dass der Positionsabstand POSA zwischen den beiden Positionssensoren PS1 und PS2 eine von einer auf die Komponente einwirkenden manuellen Bedienkraft abhängige Größe ist. Mit anderen Worten geht die Erfindung davon aus, dass die bewegbare Komponente elastisch verformbar ist und die Änderung des Positionsabstands POSA auf dieser elastischen Verformbarkeit basiert.

Anhand des ermittelten Positionsabstands POSA stellt die Erfindung im weiteren Verfahrensverlauf einen Zusammenhang zwischen dem ermittelten Positionsabstand POSA und der einwirkenden manuellen Bedienkraft her.

Entsprechend wird in einem weiteren Verfahrensschritt S30 ein Steuersignal SIG für eine Antriebseinheit AE der Komponente 30 basierend auf dem ermittelten Positionsabstand POSA erzeugt. Das heißt, es wird ein Steuersignal zur Bewegungssteuerung der Komponente 30 erzeugt, welches der über den ermittelten Positionsabstand ermittelten manuellen Krafteinwirkung entspricht.

Der Schritt S21 kann in Ausführungen neben dem Ermitteln des Positionsabstandes POSA auch ein Zuordnen einer auf die Komponente wirkenden Verstellkraft VK zu dem ermittelten Positionsabstand POSA umfassen. Dann erfolgt das Erzeugen des Steuersignals SIG für die Antriebseinheit AE basierend auf der zugeordneten Verstellkraft VK. Hierbei wird durch die Steuereinheit SE explizit ein Wert für die Verstellkraft VK ermittelt und dieser wird im weiteren Verfahrensverlauf zur Erzeugung des Steuersignals SIG zugrunde gelegt. Zu diesem Zweck greift die Steuereinheit SE bspw. auf einen internen Speicher zu, wo für verschiedene Positionsabstandswerte für die bewegbare Komponente 30 spezifische Werte für die Verstellkraft VK hinterlegt sind. Alternativ kann die Steuereinheit SE auch mittels einer abrufbaren Rechenvorschrift entsprechend eines ebenfalls komponentenspezifischen, funktionalen Zusammenhangs zwischen Positionsabstand POSA und Verstellkraft VK selbst einen Wert für die Verstellkraft VK berechnen.

Beide Varianten zum Erzeugen des Steuersignals SIG basierend auf dem Positionsabstand POSA oder der Verstellkraft VK sind äquivalent. Das Steuersignal SIG ist dabei als Steuersignal für die Antriebseinheit AE ausgebildet. Es ist ausgebildet, eine motorische Bewegungsunterstützung und/oder eine Reibungskompensation zu bewirken bzw. auszulösen.

Entsprechend wird bevorzugt in Schritt S30 das Steuersignal SIG für die Antriebseinheit SE erzeugt, indem eine Drehmomentvorgabe für die Antriebseinheit AE basierend auf dem Betrag der zugeordneten Verstellkraft VK und/oder des ermittelten Positionsabstands POSA zur motorischen Kraftunterstützung bestimmt wird. Eine Reibungskompensation kann im Gegensatz dazu bereits auf einer Richtungsinformation, also dem Vorzeichen, des Positionsabstands POSA bzw. der Verstellkraft VK mittels eines Hilfsantriebs der Antriebseinheit AE durchgeführt werden. Dies ist insbesondere vorteilhaft für Situationen, bei denen die Verstellkraft VK nicht genau ermittelt werden kann. In solchen Situationen kann die Antriebseinheit AE dennoch genutzt werden.

Um den Positionsabstand POSA bzw. die Verstellkraft VK so exakt wie möglich zu ermitteln, werden die Schritte S11 und S12 zum Erfassen des ersten und des zweiten Positionsparameters POS1, POS2 bevorzugt gleichzeitig ausgeführt. Je kleiner der zeitliche Versatz zwischen dem Erfassen der beiden Positionsparameter POS1, POS2 ist, umso genauer repräsentiert der ermittelte Positionsabstand POSA die aktuell wirkende manuelle Bedienkraft. Die Gleichzeitigkeit wird in Ausführungen der Erfindung gewährleistet, indem die Positionsparameter POS1, POS2 bspw. über den Motorcontroller 23 der Antriebseinheit AE abgerufen bzw. übermittelt werden bzw. innerhalb desselben Datenpakets übersandt werden.

In einem optionalen Schritt S13 des erfindungsgemäßen Verfahrens wird neben den Positionsparametern POS1, POS2 auch ein Beschleunigungsparameter BES der Antriebseinheit AE erfasst. Der Beschleunigungsparameter BES gibt Auskunft darüber ob und mit welcher Beschleunigung sich die bewegbare Komponente 30 bewegt. Der Beschleunigungsparameter BES zeigt folglich den Bewegungszustand der bewegbaren Komponente 30 an. Der Beschleunigungsparameter BES wird bevorzugt mittels einer an der Antriebseinheit AE der Komponente 30 angeordneten Beschleunigungssensors BS erfasst. Der Beschleunigungsparameter BES zeigt folglich einen auf die Antriebseinheit AE zurückzuführenden Bewegungsanteil der Komponente 30 an. Auch Schritt S13 erfolgt vorteilhaft gleichzeitig zum Erfassen des ersten und des zweiten Positionssensors POS1, POS2.

Weiter wird in einem weiteren optionalen Schritt S22 ein motorischer Krafteintrag MOTK der Antriebseinheit AE basierend auf dem Beschleunigungsparameter BES ermittelt. Dazu wird anhand der bewegten Masse der Komponente 30, die dem erfindungsgemäßen System bekannt ist, bspw. abrufbar im Speicher hinterlegt ist und der wirkenden Beschleunigung die Motorkraft bestimmt. Um die aktuell wirkende manuelle Bedienkraft genau zu bestimmten, wird der motorische Krafteintrag MOTK in einem ebenfalls optionalen Schritt S23 dazu verwendet, die Verstellkraft VK zu korrigieren. Die Korrektur erfolgt, indem der motorische Krafteintrag MOTK von der Verstellkraft VK abgezogen wird. Bei der Subtraktion werden Beträge und Vorzeichen der verrechneten Kräfte berücksichtigt.

In einem weiteren optionalen Schritt S24ist vorgesehen, einen vorab definierten, für die bewegbare Komponente 30 der medizinischen Anlage 10 spezifischen Offset O repräsentierend eine nicht-elastische Verformbarkeit der Komponente 30 zu subtrahieren, wenn sich das Vorzeichen des Positionsabstands PO-SA zwischen zwei Verfahrensdurchläufen ändert. Über diesen optionalen Schritt wird die Bestimmung der aktuell eingebrachten manuellen Bedienkraft noch verbessert, denn durch den Offset berücksichtigt die Erfindung weiter nicht-elastische Verformungsanteile der Komponente 30, die ebenfalls durch die manuelle Bedienkraft verursacht werden können. Der Offset liegt bspw. wieder abrufbar als vorab festgelegter und experimentell bestimmbarer, für jede Komponente spezifischer Offset-Wert in einer Speichereinheit vor. Die nicht-elastische Verformung stellt sich immer dann ein bzw. ist immer dann zu beobachten, wenn die bewegbare Komponente ihre Bewegungsrichtung ändert. Bei Fortsetzen der Bewegung in dieselbe Richtung bleibt die Verformung erhalten. Insofern wird der Offset O immer dann bei der Ermittlung der Verstellkraft VK berücksichtigt, wenn in einer Prüfschleife innerhalb des Schritts S21 ein Vorzeichenwechsel für den Positionsabstand POSA festgestellt wurde. Ergibt sich keine Vorzeichenänderung zwischen zwei Verfahrensschleifen, wird Schritt S24 nicht ausgeführt. Der Offset kann in bevorzugter Ausführung als Wert repräsentierend eine Längenänderung der Komponente 30 in einer betrachteten Raumachse ausgebildet sein.

In einem weiteren Schritt S40 wird das erzeugte Steuersignal SIG an die Antriebseinheit AE, insbesondere bspw. über einen Motorcontroller 23 der Antriebseinheit AE an diese zur Ausführung übermittelt.

Im Sinne einer kontinuierlichen Bewegungssteuerung wird das erfindungsgemäße Verfahren wiederholt mit einem zeitlichen Abstand Δt ausgeführt. Das heißt, die Positionen der Positionssensoren PS1, PS2, die von der Antriebseinheit AE ausgeübte motorische Beschleunigung und/oder dergleichen werden über einen Zeitraum, bspw. über die gesamte Betriebsdauer der medizinischen Anlage hinweg, überwacht und für die Bewegungssteuerung ausgewertet. In bevorzugter Ausführung erfolgt das Erfassen der Positionsparameter mit einem zeitlichen Abstand von 3 ms bis 15 ms, besonders bevorzugt erfolgt das Erfassen der Positionsparameter POS1, POS2 mit einem zeitlichen Abstand von 5 ms bis 10 ms. Mit diesen Zeitabständen konnte in Versuchen ein besonders intuitiver und unmittelbarer Bedieneindruck für den Nutzer geschaffen werden.

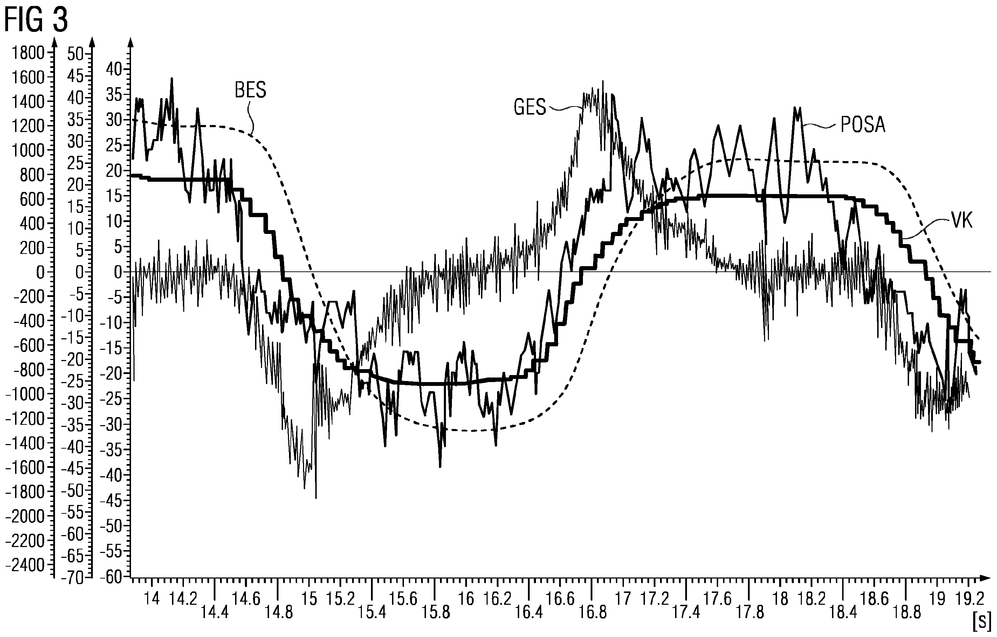

**Figur** 3 zeigt in diesem Zusammenhang ein Diagramm darstellend einen zeitlichen Verlauf des Positionsabstands POSA im Zusammenhang mit der kontinuierlich daraus ermittelten Verstellkraft VK. Der Betrachtungszeitraume erstreckt sich ca. über 5 s. Dabei wurde eine motorische Beschleunigung bzw. der daraus resultierende motorische Krafteintrag MOTK sowie ein komponentenspezifischer Offset bei Bewegungsrichtungsumkehr berücksichtigt. Ebenfalls im Diagramm mit aufgetragen ist die Bewegungsgeschwindigkeit der Komponente 30. Es zeigt sich, dass die Verstellkraft VK mit leichtem Versatz dem Verlauf des (mittleren) Positionsabstands POSA folgt.

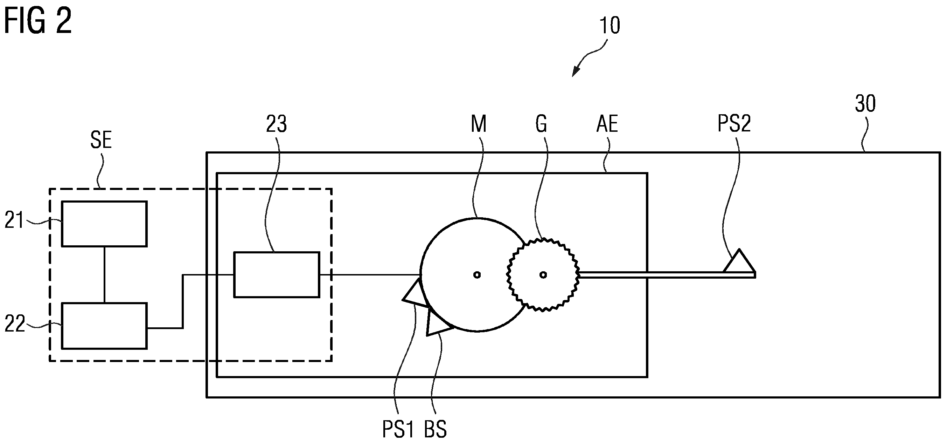

**Figur** 2 zeigt eine Detailansicht einer medizinischen Anlage 10 umfassend ein erfindungsgemäßes System zur Steuerung in einer Ausführung. Die medizinische Anlage umfasst eine bewegbare oder bewegliche Komponente 30. Die medizinische Anlage 10 ist in bevorzugter Ausführung als medizinische Bildgebungsanlage ausgebildet, d.h., sie dient der Erzeugung von medizinischen Bilddaten eines Patienten. Besonders bevorzugt handelt es sich bei der medizinischen Anlage 10 um eine medizinische Röntgenbildgebungsanlage, die eine Röntgenstrahlenquelle und einen Röntgenstrahlendetektor umfasst (nicht dargestellt). Speziell handelt es sich bei der medizinischen Anlage um eine Radiographie-Anlage, mit welcher klassische Röntgentransmissionsaufnahmen erzeugt werden. In diesen Ausführungen ist die bewegbare Komponente 30 der medizinischen Anlage 10 bevorzugt als bodenmontiertes oder deckengehängtes Stativ zum Tragen einer Bildgebungseinheit wir der Röntgenstrahlenquelle oder des Röntgenstrahlendetektors ausgebildet.

Die medizinische Anlage kann aber auch andersartig ausgebildet sein, bspw. als Anlage zur Behandlung oder Intervention. In anderen Ausführungen der medizinischen Anlage 10 kann die bewegbare Komponente 30 bevorzugt als Patiententisch ausgebildet sein, welcher zur Lagerung bzw. Positionierung des Patienten ausgebildet ist.

Die medizinische Anlage umfasst weiter eine Antriebseinheit AE sowie ein erfindungsgemäßes System zur Steuerung einer Bewegung der Komponente 30.

Die Antriebseinheit AE ist vorliegend als Teil der Komponente 30 ausgebildet. Die Antriebseinheit AE wird in Ausführungen der Erfindung bei der Bewegung der Komponenten zumindest teilweise mitbewegt. Die Antriebseinheit AE umfasst als Antriebselement hier einen als Servomotor ausgebildeten Elektromotor M, der bei Betrieb über ein Getriebe G umfassend wenigstens ein in eine Zahnstange eingreifendes Zahnrad eine Rotationsbewegung seiner Antriebswelle in eine Translationsbewegung der Komponente bewirkt. In Ausführungen kann die Antriebseinheit AE weiter einen Hilfsantrieb (nicht dargestellt) umfassen, der für eine richtungsabhängige Reibungskompensation genutzt wird.

Das System zur Steuerung einer Bewegung der Komponente 30 ist ausgebildet, die Schritte S11 bis S40 des erfindungsgemäßen Verfahrens, insbesondere auch die optionalen Schritte, wie mit Bezug zur Figur 1 oben beschrieben, auszuführen. Das System zur Steuerung einer Bewegung der Komponente 30 umfasst einen ersten an der Komponente 30 angeordneten Positionssensor PS1. Dieser ist eingerichtet, einen ersten Positionsparameters POS 1 zu erfassen. Weiter umfasst es einen zweiten an der Komponente angeordneten Positionssensor PS2, der eingerichtet ist, einen zweiten Positionsparameter POS2 zu erfassen.

Der erste Positionssensor PS1 ist hier als Lage- bzw. Drehgeber PS1 des Elektromotors M ausgebildet und insofern standardmäßig in medizinischen Anlagen mit verbaut. Der zweite Positionssensor PS2 ist vorliegend als Positionssensor ausgebildet, welcher am abtriebsseitigen Ende der Zahnstange montiert ist.

Grundsätzlich kann die Genauigkeit bei der Bestimmung des Positionsabstandes POSA bzw. der Verstellkraft VK erhöht werden, wenn die die beiden Positionssensoren einen größtmöglichen, also maximalen Ruheabstand zueinander aufweisen, also entlang der betrachteten Raumachse so weit wie möglich voneinander entfernt an der Komponente 30 angebracht sind. Derart kann das elastische Verformungsverhalten der Komponente 30 bestmöglich über den Positionsabstand abgebildet werden.

Das System umfasst ferner einen Beschleunigungssensor BS, der ebenfalls direkt am Motor M der Antriebseinheit AE verbaut ist und einen Beschleunigungsparameter BES des Motors M erfasst.

Das System zur Bewegungssteuerung umfasst ferner eine Steuereinheit SE. Diese ist eingerichtet, den Positionsabstand POSA für die Positionssensoren PS1, PS2 basierend auf dem ersten und dem zweiten Positionsparameter, POS1, POS2 zu ermitteln und ein Steuersignal SIG für die Antriebseinheit AE, insbesondere den Motor M basierend auf dem ermittelten Positionsabstand POSA zu erzeugen.

Zu diesem Zweck kann die Steuereinheit SE eine Auswerteeinheit 21 umfassen. Diese ist ausgebildet, alle Rechen-, Bestimmungs-, Ermittlungsschritte des erfindungsgemäßen Verfahrens im Sinne von S21, S22, S23, S24 und S30 auszuführen.

Die Steuereinheit SE umfasst ferner eine Datenschnittstelle zur bidirektionalen Kommunikation zwischen verschiedenen Einheiten der medizinischen Anlage 10 und der Steuereinheit SE.

Die Auswerteeinheit 21 ist ausgebildet, über Schnittstelle 22 einen Datenaustausch zum Empfang von Positions- und Beschleunigungsparametern POS1, POS2, BES sowie zur Ausgabe von Steuersignalen SIG durchzuführen. Insbesondere kann die Schnittstelle 22 eine Datenverbindung zwischen Auswerteeinheit und dem Motorcontroller 23 der Antriebseinheit AE herstellen. Der Motorcontroller 23 dient erfindungsgemäß als zentrale Kommunikations- bzw. Überwachungseinheit für die Antriebseinheit und sorgt bspw. für eine i.W. zeitgleiche Übermittlung der Positionsparameter POS1, POS2 pro Verfahrensdurchlauf.

Zudem kann der Motorcontroller 23 auch dazu dienen, auch den Beschleunigungsparameter BES zeitgleich an die Auswerteinheit 21 zu übermitteln.

Hier nicht gezeigt, jedoch im Sinne der Erfindung, kann die Komponente 30 der medizinischen Anlage 10 ausgebildet sein, nicht nur entlang einer, sondern mehrerer Raumachsen bewegt zu werden. In diesem Sinne kann das System zur Bewegungssteuerung für jede Raumachse ein Paar aus erstem und zweitem Positionssensor umfassen, wobei die Steuereinheit SE dann eingerichtet ist, einen Positionsabstand für die Positionssensor-Paare jeder Raumachse und ein jeweiliges Steuersignal für die Antriebseinheit jeder Raumachse basierend auf dem jeweiligen Positionsabstand zu erzeugen. Mit anderen Worten ist die Steuereinheit ausgebildet, das erfindungsgemäße Verfahren in Bezug auf mehrere Raumachsen, insbesondere auch zeitgleich bzw. zeitlich überlappend auszuführen. Die Schnittstelle 22 ist entsprechend ausgebildet, einen Datenaustausch zwischen der Auswerteeinheit 21 sowie einem Motorcontroller 23 der für die mehreren Raumachsen/Bewegungsrichtungen vorgesehenen Antriebseinheiten AE bereitzustellen.

Im Folgenden werden die Vorteile der Erfindung kurz zusammengefasst:
Die Erfindung ermöglicht das betrags- und richtungsmäßige Erfassen einer manuellen Bedienkraft, ohne, dass dafür ein Kraftsensor in der medizinischen Anlage vorgesehen werden muss. Die Kosten für den Kraftsensor können entfallen. Zudem ermöglicht die vorliegende Erfindung, dass die manuelle Bedienkraft an einer beliebigen Stelle in die bewegbare Komponente eingebracht wird, wodurch sich die Flexibilität und Intuitivität bei der Bedienung der medizinischen Anlage erhöht. Die Bedienung ist nun nicht mehr auf den Bereich der Komponente beschränkt, in welchem der Kraftsensor montiert ist, bspw. einem Bediengriff oder -hebel.

In Ausführungen der Erfindung wird der erste Positionsparameter mittels einer Sensorik erfasst, die standardmäßig in der Antriebseinheit verbaut ist, bspw. dem Lage- bzw. Drehgeber eines Elektromotors. Da eine medizinische Anlage typischerweise erstfehlersicher ausgeführt werden muss und dementsprechend neben dem eigentlichen Steuerpfad einen Schutzpfad zur automatischen Kontrolle und Überwachung des Steuerpfades umfasst, kann auch der zweite Positionsparamater mit standardmäßig verbauter, redundanter Schutzpfad-Sensorik erfasst werden. Zusatzkosten für die Implementierung des erfindungsgemäßen Systems zur Bewegungssteuerung können dadurch vorteilhaft geringgehalten werden.

Die Implementierung eines kleinen Hilfsantriebs, der nur für eine Reibungskompensation zur Unterstützung einer dann i.W. manuellen Verstellbewegung der Komponente spart Kosten gegenüber Servoantrieben, die die Verstellbewegung vollständig übernehmen. Gibt die Auswerteeinheit ein Steuersignal umfassend eine Drehmoment-Vorgabe für den Motor der Antriebseinheit aus, wird das System zur Bewegungsteuerung also im Drehmoment-Modus betrieben, kann zudem ein synthetisches Bediengefühl für den Nutzer vermieden werden, wie es bspw. bei einer Positions- oder Geschwindigkeits-Regelung entstehen würde.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Verfahren zur Steuerung einer Bewegung einer Komponente (30) einer medizinischen Anlage (10), wobei die Komponente (30) bewegbar ist und die bewegbare Komponente (30) als elastisches, reversibel verformbares Element ausgebildet ist, wobei die elastische Verformung der Komponente (30) durch bzw. im Wesentlichen durch eine manuell eingebrachte Bedienkraft eines Nutzers hervorgerufen wird, umfassend die Schritte:
- Erfassen (S11) eines ersten Positionsparameters (POS1) mittels eines ersten an der Komponente angeordneten Positionssensors (PS1),
- Erfassen (S12) eines zweiten Positionsparameters (POS2) mittels eines zweiten an der Komponente angeordneten Positionssensors (PS2),
- Ermitteln eines Positionsabstands (POSA) für die Positionssensoren basierend auf dem ersten und dem zweiten Positionsparameter mittels einer Steuereinheit (SE), wobei der Positionsabstand (POSA) der Abstand zwischen den beiden Positionssensoren (PS1, PS2) entlang einer Raumachse ist,
- Erzeugen eines Steuersignals (SIG) für eine Antriebseinheit (AE) der Komponente basierend auf dem ermittelten Positionsabstand.

2. Verfahren nach Anspruch 1, ferner umfassend
- Zuordnen einer auf die Komponente wirkenden Verstellkraft (VK) zu dem ermittelten Positionsabstand,
wobei das Erzeugen des Steuersignals für die Antriebseinheit basierend auf der zugeordneten Verstellkraft erfolgt.

3. Verfahren nach Anspruch 2, wobei das Erzeugen des Steuersignals für die Antriebseinheit umfasst, eine Drehmoment-vorgabe für die Antriebseinheit basierend auf dem Betrag der zugeordneten Verstellkraft zur motorischen Kraftunterstützung zu bestimmen.

4. Verfahren nach einem der Ansprüche 1 bis **3,** wobei das Erfassen des ersten und des zweiten Positionsparameters gleichzeitig erfolgt.

5. Verfahren nach einem der vorherigen Ansprüche, ferner umfassend:
- Erfassen (S13) eines Beschleunigungsparameters (BES) der Antriebseinheit zeitgleich zum Erfassen des ersten und des zweiten Positionsparameters mittels eines Beschleunigungssensors (BS), und
- Ermitteln (S22) eines motorischen Krafteintrags (MOTK) der Antriebseinheit basierend auf dem Beschleunigungsparameter,
- Korrigieren (S23) der Verstellkraft basierend auf dem motorischen Krafteintrag.

6. Verfahren nach Anspruch 5, wobei die Verstellkraft durch Subtraktion des motorischen Krafteintrags korrigiert wird.

7. Verfahren nach Anspruch 1, wobei das Erzeugen des Steuersignals für die Antriebseinheit umfasst, basierend auf dem Vorzeichen des Positionsabstands eine Drehmomentvorgabe für die Antriebseinheit zur richtungsabhängigen Reibungskompensation zu erzeugen.

8. Verfahren nach einem der vorherigen Ansprüche, wobei das Verfahren wiederholt mit einem zeitlichen Abstand (Δt) ausgeführt wird.

9. Verfahren nach Anspruch 8, wobei das Ermitteln des Positionsabstands weiter umfasst, einen vorab definierten, für die bewegbare Komponente der medizinischen Anlage spezifischen Offset (O) repräsentierend eine nicht-elastische Verformbarkeit der Komponente zu subtrahieren, wenn sich das Vorzeichen des Positionsabstands zwischen zwei Verfahrensdurchläufen ändert.

10. System zur Steuerung einer Bewegung einer Komponente (30) einer medizinischen Anlage (10), umfassend:
- eine bewegbare Komponente (30) , welche als elastisches, reversibel verformbares Element ausgebildet ist, wobei die elastische Verformung der Komponente (30) durch bzw. im Wesentlichen durch eine manuell eingebrachte Bedienkraft eines Nutzers hervorgerufen wird,
- einen ersten an der Komponente angeordneten Positionssensor (PS1), eingerichtet zum Erfassen eines ersten Positionsparameters (POS1),
- einen zweiten an der Komponente angeordneten Positionssensor (PS2), eingerichtet zum Erfassen eines zweiten Positionsparameters (POS2),
- eine Steuereinheit (SE), eingerichtet
o zum Ermitteln eines Positionsabstands (POSA) für die Positionssensoren basierend auf dem ersten und dem zweiten Positionsparameter, wobei der Positionsabstand (POSA) der Abstand zwischen den beiden Positionssensoren (PS1, PS2) entlang einer Raumachse ist, und
o zum Erzeugen eines Steuersignals (SIG) für eine Antriebseinheit (AE) der Komponente basierend auf dem ermittelten Positionsabstand.

11. System nach Anspruch 10, wobei der erste und der zweite Positionssensor derart an der Komponente angeordnet sind, dass sie einen maximalen Ruheabstand entlang einer Raumachse zueinander aufweisen.

12. System nach Anspruch 10 oder 11, umfassend ein Paar aus erstem und zweitem Positionssensor für jede Raumachse, wobei die Steuereinheit eingerichtet ist, einen Positionsabstand für die Positionssensor-Paare jeder Raumachse und ein jeweiliges Steuersignal für die Antriebseinheit jeder Raumachse basierend auf dem jeweiligen Positionsabstand zu erzeugen.

13. System nach einem der Ansprüche 10 bis 12, wobei jeweils der erste Positionssensor als Drehgeber der Antriebseinheit ausgebildet ist.

14. Medizinische Anlage (10) umfassend eine Antriebseinheit (AE) sowie ein System zur Steuerung einer Bewegung einer Komponente nach einem der Ansprüche 10 bis 13.

15. Medizinische Anlage nach Anspruch 14, wobei die bewegbare Komponente als Stativ zum Tragen einer Bildgebungseinheit oder als Patiententisch ausgebildet ist.

## Claims

1. Method for controlling a motion of a component (30) of a medical installation (10), wherein the component can be moved and the moveable component (30) is designed as a resilient, reversibly deformable element, wherein the resilient deformation of the component (30) is brought about or is substantially brought about by a manual application of operating force by a user, comprising the steps:
- capture (S11) of a first position parameter (POS1) by means of a first position sensor (PS1) arranged on the component,
- capture (S12) of a second position parameter (POS2) by means of a second position sensor (PS2) arranged on the component,
- determination of a position spacing (POSA) for the position sensors on the basis of the first and the second position parameter by means of a control unit (SE), wherein the position spacing (POSA) is the spacing between the two position sensors (PS1, PS2) along a spatial axis,
- generation of a control signal (SIG) for a drive unit (AE) of the component on the basis of the determined position spacing.

2. Method according to claim **1,** further comprising
- assignment of an adjusting force (VK) acting on the component to the determined position spacing,
wherein the generation of the control signal for the drive unit takes place on the basis of the assigned adjusting force.

3. Method according to claim 2, wherein the generation of the control signal for the drive unit comprises ascertaining a torque specification for the drive unit on the basis of the absolute value of the assigned adjusting force for motorised power assistance.

4. Method according to one of claims 1 to **3,** wherein the capture of the first and the second position parameter takes place simultaneously.

5. Method according to one of the preceding claims, further comprising:
- capture (S13) of an acceleration parameter (BES) of the drive unit simultaneously with the capture of the first and the second position parameter by means of an acceleration sensor (BS), and
- determination (S22) of a motorised input of force (MOTK) of the drive unit on the basis of the acceleration parameter,
- correction (S23) of the adjusting force on the basis of the motorised input of force.

6. Method according to claim 5, wherein the adjusting force is corrected by subtraction of the motorised input of force.

7. Method according to claim 1, wherein the generation of the control signal for the drive unit comprises generating, on the basis of the sign of the position spacing, a torque specification for the drive unit for direction-dependent friction compensation.

8. Method according to one of the preceding claims, wherein the method is carried out repeatedly with a time interval (Δt).

9. Method according to claim 8, wherein the determination of the position spacing further comprises subtracting a previously defined offset (O) specific to the movable component of the medical installation and representing a non-elastic deformability of the component if the sign of the position spacing changes between two passes of the method.

10. System for controlling a motion of a component (30) of a medical installation (10), comprising:
- a moveable component (30) which is designed as a resilient, reversibly deformable element, wherein the resilient deformation of the component (30) is brought about or is substantially brought about by a manual application of operating force by a user,
- a first position sensor (PS1) arranged on the component, designed for the capture of a first position parameter (POS1),
- a second position sensor (PS2) arranged on the component, designed for the capture of a second position parameter (POS2),
- a control unit (SE), designed
o for the determination of a position spacing (POSA) for the position sensors on the basis of the first and the second position parameter, wherein the position spacing (POSA) is the spacing between the two position sensors (PS1, PS2) along a spatial axis, and
o for the generation of a control signal (SIG) for a drive unit (AE) of the component on the basis of the determined position spacing.

11. System according to claim 10, wherein the first and the second position sensor are arranged on the component such that they have a maximum spacing at rest to one another along a spatial axis.

12. System according to claim 10 or 11, comprising a pair consisting of a first and second position sensor for each spatial axis, wherein the control unit is designed to generate a position spacing for the position sensor pairs of each spatial axis and a respective control signal for the drive unit of each spatial axis on the basis of the respective position spacing.

13. System according to one of claims 10 to 12, wherein in each case the first position sensor is designed as a rotary encoder of the drive unit.

14. Medical installation (10) comprising a drive unit (AE) and a system for controlling a motion of a component according to one of claims 10 to 13.

15. Medical installation according to claim 14, wherein the movable component is designed as a stand to support an imaging unit or as a patient table.

## Revendications

1. Procédé de commande **d'un** déplacement **d'un** composant (30) d'une installation (10) médicale, dans lequel le composant (30) est mobile et le composant (30) mobile est constitué sous la forme d'un élément élastique déformable de manière réversible, dans lequel la déformation élastique du composant (30) est provoquée par ou essentiellement par une force de manœuvre appliquée manuellement par un utilisateur, comprenant les stades :
- détection (S11) d'un premier paramètre (POS1) de position au moyen d'un premier capteur (PS1) de position monté sur le composant,
- détection (S12) d'un deuxième paramètre (POS2) de position au moyen d'un deuxième capteur (PS2) de position monté sur le composant,
- détermination d'une distance (POSA) de position des capteurs de position sur la base du premier et du deuxième paramètres de position au moyen d'une unité (SE) de commande, dans lequel la distance (POSA) de position est la distance entre les deux capteurs (PS1, PS2) de position suivant un axe dans l'espace,
- production d'un signal (SIG) de commande d'une unité (AE) d'entraînement du composant sur la base de la distance de position déterminée.

2. Procédé suivant la revendication 1, comprenant en outre
- affectation d'une force (VK) de déplacement s'appliquant au composant à la distance de position déterminée,
dans lequel la production du signal de commande de l'unité d'entraînement s'effectue sur la base de la force de déplacement affectée.

3. Procédé suivant la revendication 2, dans lequel la production du signal de commande de l'unité d'entraînement comprend une prescription de couple pour l'unité d'entraînement sur la base de la valeur absolue de la force de déplacement affectée pour la détermination d'une assistance de force motorisée.

4. Procédé suivant l'une des revendications 1 à **3,** dans lequel la détection du premier et du deuxième paramètres de position s'effectue en même temps.

5. Procédé suivant l'une des revendications précédentes, comprenant en outre :
- détection (S13) d'un paramètre (BES) d'accélération de l'unité d'entraînement en même temps que la détection du premier et du deuxième paramètres de position au moyen d'un capteur (BS) d'accélération, et
- détermination (S22) d'une application (MOTK) de force motorisée à l'unité d'entraînement sur la base du paramètre d'accélération,
- correction (S23) de la force de déplacement sur la base de l'application de force motorisée.

6. Procédé suivant la revendication 5, dans lequel la force de déplacement est corrigée par soustraction de l'application de force motorisée.

7. Procédé suivant la revendication 1, dans lequel la production du signal de commande de l'unité d'entraînement comprend, sur la base du signe de la distance de position, une prescription de couple pour l'unité d'entraînement pour produire la compensation de frottement en fonction de la direction.

8. Procédé suivant l'une des revendications précédentes, dans lequel on effectue le procédé de manière répétée avec une distance (Δt) dans le temps.

9. Procédé suivant la revendication 8, dans lequel la détermination de la distance de position comprend en outre soustraire un décalage (O) défini à l'avance, spécifique au composant mobile de l'installation médicale et représentant une déformabilité non élastique du composant, lorsque le signe de l'écart de position entre deux passes du procédé se modifie.

10. Système de commande d'un mouvement d'un composant (30) d'une installation (10) médicale, comprenant :
- un composant (30) mobile, qui est constitué sous la forme d'un élément élastique déformable de manière réversible, dans lequel la déformation élastique du composant (30) est provoquée par ou essentiellement par une force appliquée manuellement par un utilisateur,
- un premier capteur (PS1) de position monté sur le composant et agencé pour la détection d'un premier paramètre (POS1) de position,
- un deuxième capteur (PS2) de position monté sur le composant et agencé pour la détection d'un deuxième paramètre (POS2) de position,
- une unité (SE) de commande, agencée
o pour la détermination d'une distance (POSA) de position des capteurs de position sur la base du premier et du deuxième paramètres de position, dans lequel la distance (POSA) de position est la distance entre les deux capteurs (PS1, PS2) de position suivant un axe dans l'espace, et
o pour la production d'un signal (SIG) de commande d'une unité (AE) d'entraînement du composant sur la base de la distance de position déterminée.

11. Système suivant la revendication 10, dans lequel le premier et le deuxième capteurs de position sont montés sur le composant, de manière à avoir entre eux une distance maximum au repos suivant un axe dans l'espace.

12. Système suivant la revendication 10 ou 11, comprenant une paire de premier et de deuxième capteurs de position pour chaque axe dans l'espace, dans lequel l'unité de commande est agencée pour produire une distance de position pour les paires de capteurs de position de chaque axe dans l'espace et un signal de commande respectif de l'unité d'entraînement pour chaque axe dans l'espace sur la base de la distance de position respective.

13. Système suivant l'une des revendications 10 à 12, dans lequel respectivement le premier capteur de position est constitué sous la forme d'un codeur rotatif de l'unité d'entraînement.

14. Installation (10) médicale comprenant
une unité (AE) d'entraînement ainsi qu'un système de commande d'un mouvement d'un composant suivant l'une des revendications 10 à 13.

15. Installation médicale suivant la revendication 14, dans lequel le composant mobile est constitué sous la forme d'un support pour porter une unité d'imagerie ou sous la forme d'une couchette de patient.
